# EUROPEAN PATENT SPECIFICATION

(11) **EP 0 967 921 B1**
(45) Date of publication and mention of the grant of the patent: **15.05.2013**
(21) Application number: 97950876.9
(22) Date of filing: 03.12.1997
(51) Int. Cl.: A61B 17/04

(54) **SUTURE ANCHOR**
NÄHFADENANKER
DISPOSITIF DE FIXATION DE SUTURE

(30) Priority: 19.12.1996 FI 965111
(43) Date of publication of application: 05.01.2000
(73) Proprietor: Linvatec Biomaterials Oy, 33721 Tampere (FI)
(72) Inventor: ANDERSON, David, W., Villanova, PA 19085 (US); HELEVIRTA, Pertti, FIN-33710 Tampere (FI); PIRHONEN, Eija, FIN-33710 Tampere (FI); POHJONEN, Timo, FIN-33720 Tampere (FI); TAMMINMÄKI, Markku, FIN-33400 Tampere (FI); TÖRMÄLÄ, Pertti, FIN-33720 Tampere (FI)
(74) Representative: Draper, Martyn John
(86) International application number: PCT/US1997/022428
(87) International publication number: WO 1998/026717

(56) References cited:
- WO-A-95/24166
- WO-A-96/28100
- US-A- 5 258 016
- US-A- 5 324 308
- US-A- 5 423 860
- US-A- 5 464 427

## Description

The present invention relates to a suture anchor in accordance with the preamble of the accompanying Claim 1.

Suture anchors are used in surgery, in particular in orthopedic surgical operations for placing a part of the suture in and attaching it to a bone, wherein the part of the suture that is not attached, most commonly two parts projecting from the bone, can be further used e.g. for connecting detached connective tissue, such as ligament, to the bone. To place the suture anchor into a bone, a hole is made in the bone, e.g. by drill-ing, into which hole the suture anchor and a part of the suture is inserted, wherein the object of the suture anchor is to keep the suture firmly in the bone, in particular during and after the orthopedic operation.

In respect of the prevailing level of technology in the field, reference is made mainly to the following patents: US-5,156,616, US-5,472,452, US-5,037,422 and WO 96/28100.

In accordance with the suture anchor of patent US-5,156,616, two separate sutures are inserted through a hole penetrating the body of the suture anchor in the longitudinal direction and situated substantially in the middle of the cross-section of the anchor body in a manner that the other ends of the sutures extend clearly outside the end at the base side of the suture anchor and the ends at the head side of the suture anchor are tied together, wherein the knot formed thereby prevents the suture from gliding through the hole, out from the suture anchor via its base. The suture anchor is provided with screw threads.

The suture anchor in accordance with patent US-5,472,452 comprises a tubular body having a substantially quadrangular cross-sectional form, and a core part to be inserted therein. Two walls of the anchor body are provided with projecting branches which are pressed by the core part against the walls of a hole formed in the bone when the suture anchor is pressed into the hole. The branches are further provided with means which can be grabbed by a special tool, e.g. for moving the suture anchor inside the hole. The suture is arranged to circle outside the anchor body via two of its opposite vertical walls and heads. For implementation of this, the heads of said two vertical walls are provided with cuttings in which the suture is placed at the head of the anchor body.

The suture anchor of patent US-5,037,422 is composed of a conical, hollow body which is positioned by pressing or hitting in a hole made in the bone. For keeping the anchor body in its place, it is provided with two splits situated substantially at the opposite walls thereof and having a cross-section parallel with the suture anchor, wherein the anchor body is contracted when inserted in the hole, tending to expand by pressing the gearing that is formed on the outer surface of the anchor body against the walls of the hole. The suture is positioned to travel substantially through the splits, parallel with them, and arranged to circle via a substantially straight, tubular turning portion situated in the vicinity of the head of the anchor body.

The suture anchor in accordance with the prior-mentioned patent US-5,156,616 has a simple structure and comprises a screw-thread arrangement which is advantageous when placed in a bone. However, it is unsafe to keep the sutures tied in the suture anchor merely supported by said knot, and it is not possible to fully prevent the sutures from being damaged, in particular at the junction of the knot and the hole penetrating the suture anchor. Furthermore, a device installed in the base of the suture anchor for threading the suture anchor into the bone can damage the sutures, which are situated partially in a sleeve formed for the head of said device in the base of the anchor body.

The drawbacks of the two other solutions mentioned above (US-5,472,452 and US-5,037,422) are mainly related to the complexity in their structure and use. A particular drawback is that the suture channels through which the sutures are intended to be placed in the suture anchor are formed in a manner that harmfully abrupt alterations of direction take place, in particular adjacent to the heads of the suture anchors (an abrupt alteration of direction of about 90°) which easily results in partial breaking or even cutting off of the suture when the suture is tightened in a later phase of the orthopedic surgical procedure, because the suture is subjected to a strong tensile stress on its outermost surface.

The object of the invention is to introduce a suture anchor whereby the above-described shortcomings pertaining to known suture anchors can be eliminated to a great extent and thus the prevailing level of technology in the field can be elevated.

For achieving this object, the suture anchor according to the invention is characterized by a novel arrangement between the body of the suture anchor and the suture. This novel arrangement prevents the formation of abrupt alterations of course which add to the risk of damaging the suture. The body of the suture anchor consists of a single part, and the suture channel in which the suture is placed is entirely formed in the anchor body without any separate means and is composed of two longitudinal guides and a particularly curved turning portion which connects them. By means of this arrangement, in particular in view of the turning portion, the tensile strength of the suture required in the surgical operation can be maximally utilized. The primary characteristics of the suture anchor of the invention will be presented in the characterizing portion of the appended Claim 1.

The simple, substantially closed structure of the anchor body markedly adds to the strength of the suture anchor and thus also increases its operational reliability. The arrangements related to the suture channel also efficiently prevent the risk of damaging, which is present when bringing the suture anchor in the bone and which is particularly caused when the suture gets in contact with the surface of the bone of the drill canal.

The above-mentioned features and other features characteristic to the present invention, as well as embodiments of the invention will be presented in the accompanying dependent Claims.

The invention will be illustrated in more detail in the following specification, with reference made to the accompanying drawings. In the drawings,
- Figs. 1 to 8: show various embodiments of the suture anchor as seen from the side (Figs. 1, 3, 5 and 7) and corresponding cross-sections in the direction of the longitudinal axis at the location of the suture channel (Figs. 2, 4, 6 and 8), and
- Figs. 9a to d: show heads of various suture anchors used in the test arrangement illustrated as an example.

The suture anchor of the present invention comprises a substantially closed and elongated body 1 which is preferably manufactured of a single piece and has preferably a circular cross-section. The longitudinal outer surface of the anchor body 1 is advantageously provided with a set of projections, advantageously a screw thread 2 having the same size as the longitudinal side of the anchor body 1, for placing the suture anchor, in particular by threading, in a hole formed in the bone. The set of projections can optionally have e.g. a scale-like structure. In this respect reference is made to the structure described in patent FI-95537.

The anchor body 1 is preferably manufactured of some bioabsorbable material, wherein it is usually not necessary to remove it from the bone, but is it obvious that the possible removal and/or precise moving of a suture anchor provided with a screw thread 2 can be carried out in the drill canal or hole formed in the bone in a fairly easy manner. It is self-evident that the screw thread 2 does not require the suture anchor to be positioned in the bone by threading; instead it is possible to insert it into the hole e.g. simply by pressing or hitting.

The head of the anchor body 1a is preferably formed in a manner that it tapers at least to some extent towards the peak of the head. It is also advantageous that the head of the anchor body 1 is rounded. In the base 1 b of the anchor body, connecting means 6 are formed for connecting an external device with the anchor body for placing the suture anchor in the bone, wherein the connecting means can comprise e.g. a sleeve wrench or the like for the blade part.

The suture channel K for binding the suture 7 to the suture anchor is entirely formed in the anchor body 1 by using grooves, tubular holes and/or combinations thereof in order to constitute substantially U-formed arrangements. Thus, for example owing to details pertaining to manufacturing techniques and/or bringing the suture anchor in operatable condition, the suture channel K can be formed in accordance with the embodiments shown in the drawings.

The suture channel K comprises two longitudinal guides 3, 4 and a turning portion 5 connecting them at their ends of the head 1 a side of the suture anchor. The longitudinal guides 3, 4 are situated in the anchor body 1 substantially in accordance with the longitudinal direction thereof and, also, substantially on the opposite directions of the longitudinal central line of the anchor body 1. The turning portion 5 is situated at the end of the head 1a side of the longitudinal guides 3, 4 and constituting an extension thereto. It is characteristic to the invention that the turning portion 5 is preferably a continuous curved form, e.g. a circle form, wherein in the area of the suture channel K, no abrupt, sharp angles or the like are formed in the suture 7 placed in the suture channel K, at which the risk of damaging the suture would be particularly notable in situations in which the suture is subjected to a power influence, said power influence tightening the suture especially during or after a surgical operation. The effect of the form of the turning point 5 on tensile strength is illustrated at the end of the specification by means of an example describing one experimental arrangement.

Since the duration of the tensile stress on the suture can be influenced expressly by the design of the turning portion 5, in particular with the grade of curvature, it is substantial that the turning portion 5 is formed in a manner that the bending radius of the suture 7 placed therein does not cause substantial tensile stress on the outermost surface of the suture 7 when the suture is subjected to an effective force parallel with the longitudinal guides 3, 4 and directed towards the base 1b of the anchor body 1 in connection with a surgical operation. To implement this object, all the embodiments that will be described in the following share the common feature that the turning portion 5 extends in the longitudinal alignment of the suture anchor, wherein a turning point KP at the top of the turning portion 5 is located at a distance L from the junctions 5a, 5b of the longitudinal guides 3, 4 and the turning portion 5. This results in the fact that every alteration angle at the turning portion 5 is smaller than 90° or, in particular (as in the advantageous embodiments shown in the Figures) that the alteration angle of the turning portion 5 is continuous and the turning portion 5 has a curved configuration.

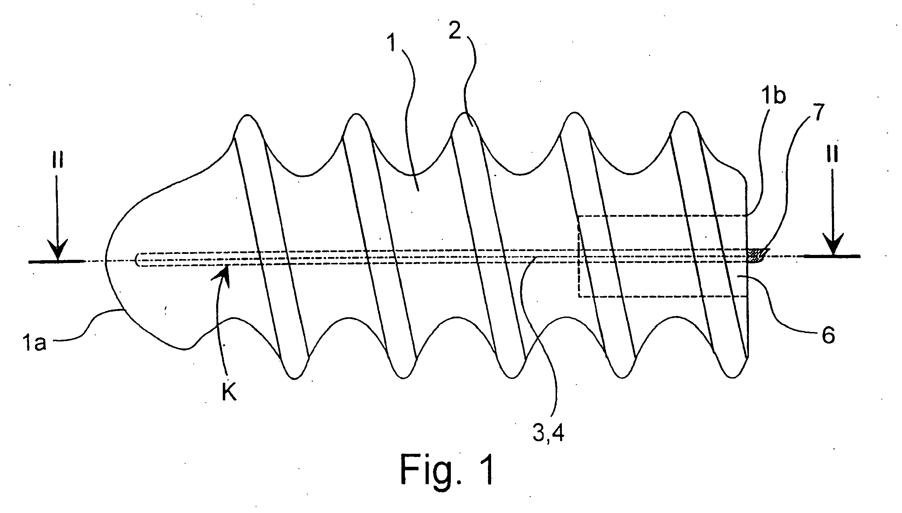

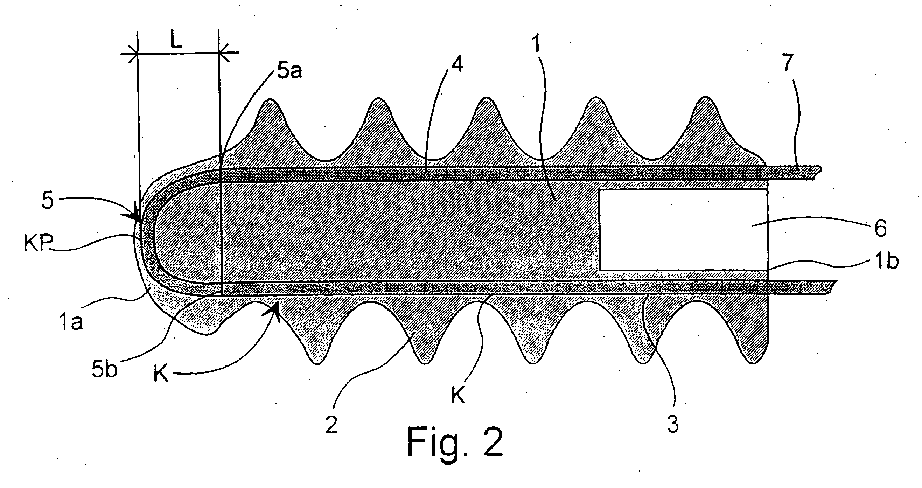

Figs. 1 and 2 show an embodiment of the suture anchor, wherein the suture channel K is formed entirely inside the anchor body 1, wherein the longitudinal guides 3, 4 are holes that are open at the base 1b of the anchor body 1, directed towards the top 1a of the anchor body, and situated substantially in alignment, preferably on the opposite sides of the longitudinal central line of the anchor body 1, adjacent to the outer surface of the anchor body 1, at the base parts of the screw thread 2. A curved, preferably circular hole forming the turning portion 5 and placed entirely inside the anchor body 1 is situated successive to the longitudinal guides 3, 4 combining their head 1 a ends at the junctions 5a, 5b.

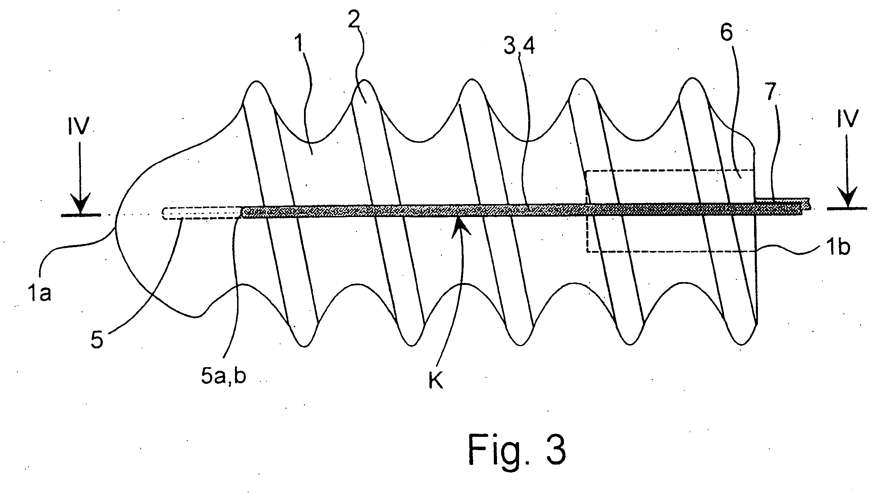

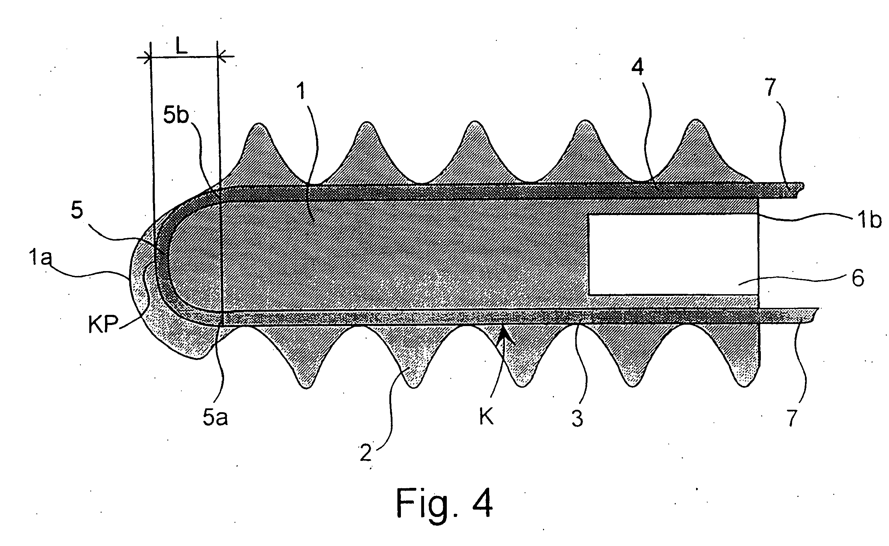

Figs. 3 and 4 show a suture anchor having longitudinal guides 3, 4 of the suture channel K which consist of two grooves made in the outer surface of the anchor body 1, said grooves being situated preferably at the opposite sides of the anchor body 1 and extending from the base 1b of the anchor body to the ends of the curved, preferably circular hole situated inside the anchor body 1 and forming the turning portion 5, wherein the junctions 5a, 5b that are situated on the outer surface of the anchor body 1 are constituted.

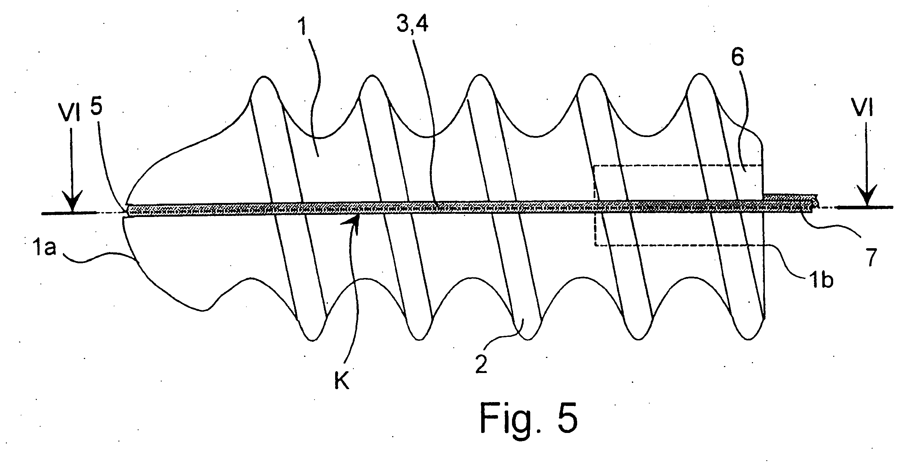

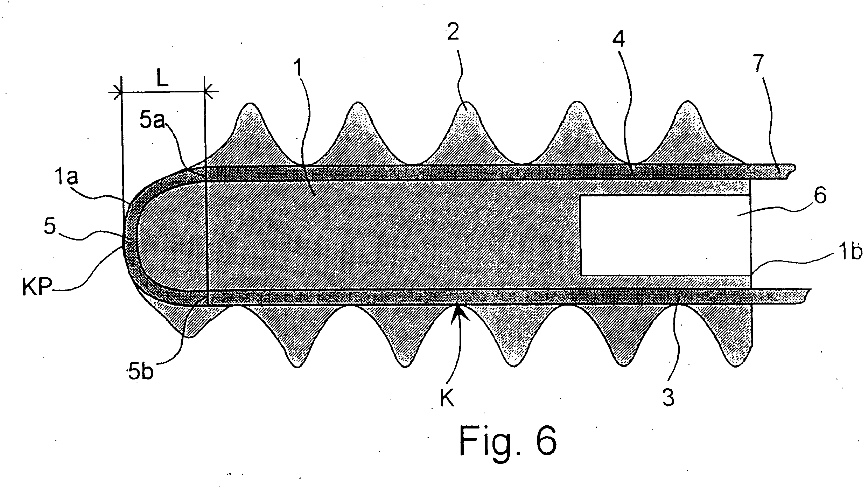

Figs. 5 and 6 illustrate an embodiment where both the longitudinal guides 3, 4 of the suture channel K and the turning portion 5 have a groove form and are situated on the surface of the anchor body 1, wherein the turning portion 5 is formed at the head 1a of the anchor body 1, on the outer surface thereof, to pass the head 1a in curved, preferably circular groove form, wherein the longitudinal guides 3, 4 connected to the ends of the groove-formed turning portion 5 at the junctions 5a, 5b are placed on the opposite sides of the anchor body 1, substantially parallel with the longitudinal direction of the anchor body 1, to extend to the base 1b of the anchor body.

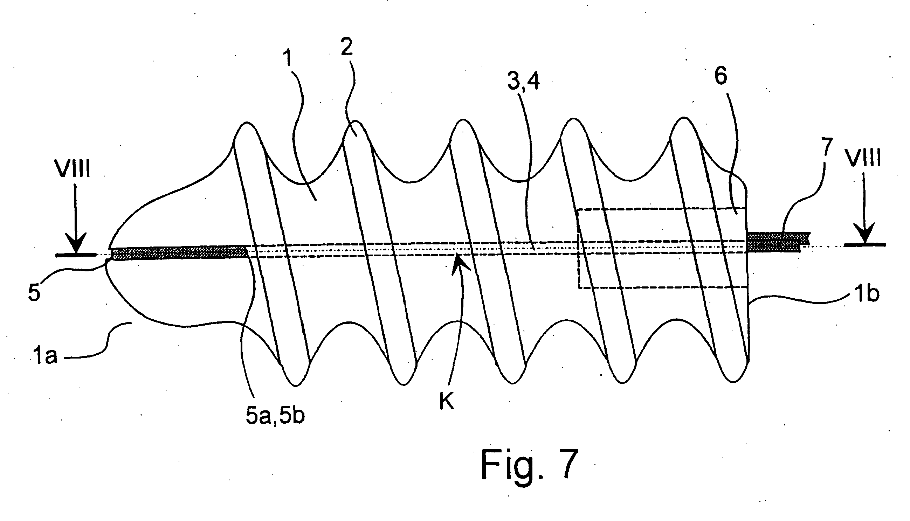

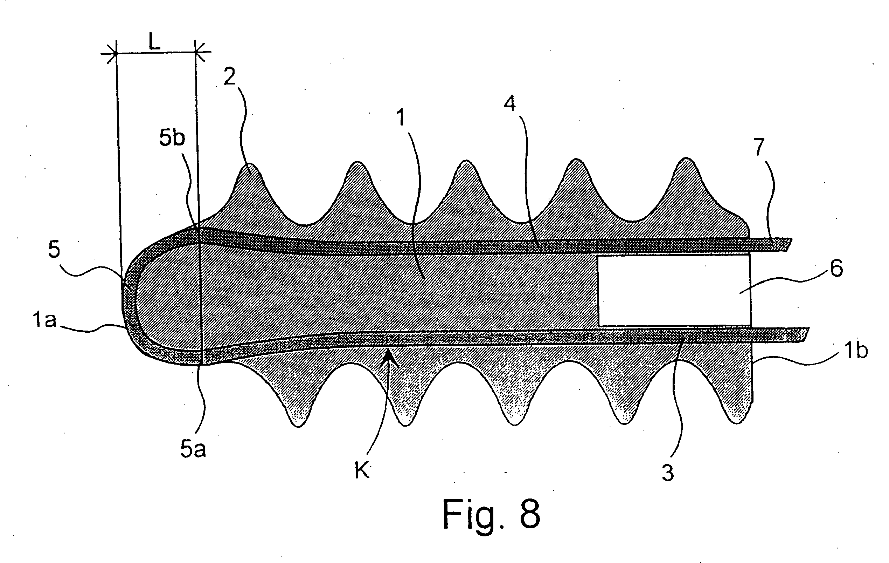

As shown in Figs. 7 and 8, the turning portion 5 of the suture channel K constitutes a groove at the head 1a of the anchor body 1, in a respective manner as in the embodiment of Fig. 3, but the longitudinal guides 3, 4 are formed inside the anchor body 1 (cf. Fig. 1) in a manner that they constitute holes which penetrate the anchor body 1 in its longitudinal direction and extend from the base 1 b to the ends of the turning portion 5, up to the junctions 5a, 5b.

It is obvious that the longitudinal guides 3, 4 can easily be placed in the base 1 b area so that the connecting means 6 can be formed between the longitudinal guides 3, 4 and the connecting means 6 can be used without causing any risk of damaging the suture 7 itself, e.g. when the suture anchor is seated into the bone.

The functionality of the invention is illustrated in the following example.

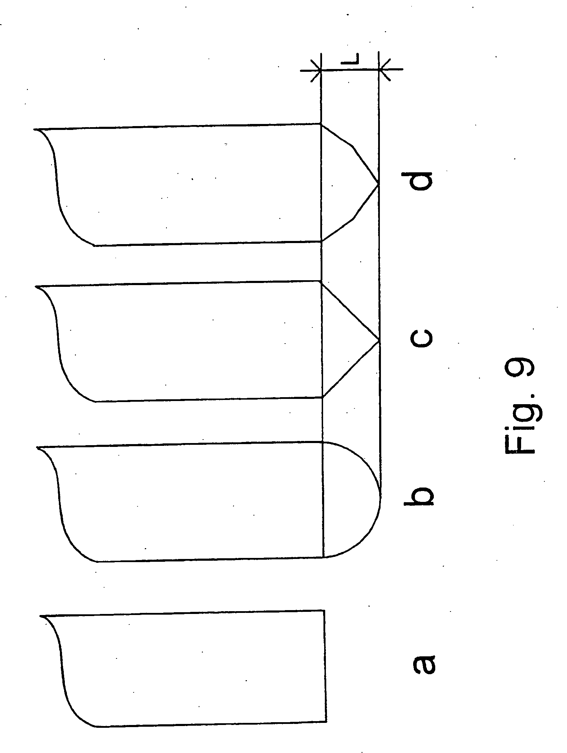

Four suture anchors (Fig. 9 a to d) of various types, in particular with regard to the form of the turning portion 5, were manufactured, wherein
- the head form a is a so-called blunt head, wherein the suture has to circle the head substantially via two abrupt angles of about 90° (cf. US-5,472,452),
- the head form b is one advantageous head form in accordance with the invention, this head form being a curved, preferably a circled form,
- the head form c is included within the scope of the inventive idea, being a pointed form, in which the suture has to circle via three alterations of course (angles e.g. 60°), and
- the head form d comprises more than three angles, e.g. five angles of 36°.

The aim of the test was to demonstrate how the form of the head of the suture anchor affects the suture Dexon® (USP 1) placed in the suture anchor. The suture anchors were attached to first jaws of a device for measuring tensile strength, and the free ends of the suture were attached to second jaws of the device, whereafter the suture was subjected to an effective force, said effective force stretching the suture for breaking it off. In the test, the following minimum effective forces were obtained for breaking off the suture:
a) 87 to 96 N
b) 186 to 210 N
c) 153 to 161 N
d) 154 to 187 N

The results indicate that the turning portion form b, which, in accordance with the invention, is the most advantageous form for the turning point in the suture anchor, gives the best results in view of the tensile strength. The options c and d improve the tensile strength substantially compared to the option a.

The invention is neither limited solely to what is described above nor to the embodiments illustrated in the Figures, but it can be modified within the scope of the inventive idea presented in the accompanying Claims.

## Claims

1. A one-piece suture anchor for anchoring a suture (7) into a bone, wherein a hole for the suture anchor is formed in the bone, the suture anchor comprising an elongated anchor body (1) having a head (1a) and a base (1b), said body (1) provided with a suture channel (K) composed of two elongated longitudinal guides (3, 4) substantially aligned with the longitudinal direction of the anchor body (1) and a turning portion (5) combining the longitudinal guides at the anchor body head (1a) side, **characterized in that** the turning portion (5) is formed substantially in a manner that it is aligned with the longitudinal dimension of the suture anchor, wherein the turning point at the head of the turning portion (5) is situated at a longitudinal distance from the junctions (5a, 5b) of the longitudinal guides (3, 4) and the turning portion (5), wherein the longitudinal guides and the turning portion have a groove form and are situated on the outer surface of the suture anchor, or the longitudinal guides are channels formed inside the anchor body.

2. A suture anchor as set forth in Claim 1, **characterized in that** the alteration angle of the turning portion (5) between the junctions (5a, 5b) is continuous and that the turning portion (5) has a curved configuration.

3. A suture anchor as set forth in Claim 1, **characterized in that** each alteration angle of the turning portion (5) between the junctions (5a, 5b) is smaller than 90°.

4. A suture anchor as set forth in any of the preceding Claims, **characterized in that** the longitudinal guides (3, 4) consist of two holes which are open at the base (1b) of the anchor body and are positioned inside the anchor body (1), said holes being connected together at their ends of the anchor body head (1a) side that remain inside the anchor body (1) by means of a tubular turning portion (5) that is situated inside the anchor body (1).

5. A suture anchor as set forth in any of Claims 1 to 3, **characterized in that** the longitudinal guides (3, 4) consist of two grooves formed on the surface of the anchor body (1) and extending parallel with the longitudinal direction of the anchor body (1) from the base (1b) to the head (1a) of the anchor body, substantially to the opposite sides of the anchor body (1), wherein the turning portion (5) is composed of the groove formed on the surface of the head (1a) of the anchor body and connecting the longitudinal guides (3, 4).

6. A suture anchor as set forth in any of Claims 1 to 3, **characterized in that** the longitudinal guides (3, 4) consist of two holes longitudinally parallel with the anchor body (1) and penetrating the anchor body, wherein the turning portion (5) is composed of a groove formed on the surface of the head (1a) of the anchor body and connecting the longitudinal guides (3, 4).

7. A suture anchor as set forth in any of the preceding Claims, **characterized in that** the outer surface of the anchor body (1) is provided with a set of projections, advantageously a screw thread (2), which is substantially of the same length as the longitudinal side of the anchor body (1).

8. A suture anchor as set forth in any of the preceding Claims, **characterized in that** the anchor body (1) is provided with connecting means (6) formed at the base (1b) thereof for connecting the suture anchor to an external device for seating the suture anchor into the bone.

9. A suture anchor as set forth in Claims 7 and 8, **characterized in that** the connecting means (6) comprise a sleeve or the like for connecting the suture anchor to a wrench or the like.

10. A suture anchor as set forth in any of the preceding Claims, **characterized in that** the anchor body (1) is manufactured of biodegradable material.

11. A suture anchor as set forth in any of the preceding Claims, **characterized in that** the suture does not extend past the head (la) of the anchor body.

## Patentansprüche

1. Einstückiger Fadenanker zum Verankern eines Fadens (7) in einem Knochen, wobei in dem Knochen ein Loch für den Fadenanker ausgebildet ist und wobei der Fadenanker einen länglichen Ankerkörper (1) mit einem Kopf (1a) und einer Basis (1b) aufweist, wobei der Körper (1) mit einem Fadenkanal (K) ausgestattet ist, der aus zwei länglichen, in Längsrichtung verlaufenden Führungen (3, 4), die in etwa mit der Längsrichtung des Ankerkörpers (1) ausgerichtet sind, und einem Umkehrabschnitt (5) zusammengesetzt ist, der die beiden in Längsrichtung verlaufenden Führungen auf der Seite des Ankerkopfes (1a) verbindet, **dadurch gekennzeichnet, dass** der Umkehrabschnitt (5) im Wesentlichen in einer solchen Weise ausgebildet ist, dass er an die Ausdehnung des Fadenankers in Längsrichtung angepasst ist, wobei sich der Umkehrpunkt am Kopf des Umkehrabschnittes (5) bezüglich der Längsrichtung in einem Abstand zu den Anschlussstellen (5a, 5b) der in Längsrichtung verlaufenden Führungen (3, 4) an den Umkehrabschnitt (5) befindet, wobei die in Längsrichtung verlaufenden Führungen und der Umkehrabschnitt nutenförmig ausgebildet sind und sich an der äußeren Oberfläche des Fadenankers befinden oder wobei die in Längsrichtung verlaufenden Führungen Kanäle bilden, die innerhalb des Fadenankers ausgebildet sind.

2. Fadenanker nach Anspruch 1, **dadurch gekennzeichnet, dass** der Änderungswinkel des Umkehrabschnitts (5) zwischen den Anschlussstellen (5a, 5b) kontinuierlich ist und der Umkehrabschnitt (5) eine Krümmung aufweist.

3. Fadenanker nach Anspruch 1, **dadurch gekennzeichnet, dass** jeder Änderungswinkel des Umkehrabschnitts (5) zwischen den Anschlussstellen (5a, 5b) kleiner als 90° ist.

4. Fadenanker nach einem der vorhergehenden Ansprüche, **dadurch gekennzeichnet, dass** die in Längsrichtung verlaufenden Führungen (3, 4) aus zwei, an der Basis (1b) des Ankerkörpers offenen Löchern bestehen und innerhalb des Ankerkörpers (1) angeordnet sind, wobei die Löcher an ihren zur Ankerkopfseite (1a) gelegenen, innerhalb des Ankerkörpers (1) verbleibenden Enden mittels eines rohrförmigen, innerhalb des Ankerkörpers (1) angeordneten Umkehrabschnitts (5) miteinander verbunden sind.

5. Fadenanker nach einem der Ansprüche 1 bis 3, **dadurch gekennzeichnet, dass** die in Längsrichtung verlaufenden Führungen (3, 4) aus zwei Nuten bestehen, die an der Oberfläche des Ankerkörpers (1) ausgebildet sind, wobei sie sich parallel zur Längsrichtung des Ankerkörpers (1) von der Basis (1b) zum Kopf (1a) des Ankerkörpers im Wesentlichen zu einander gegenüberliegenden Seiten des Ankerkörpers (1) erstrecken, wobei der Umkehrabschnitt (5) von der an der Oberfläche des Kopfes (1a) des Ankerkörpers ausgebildeten Nut gebildet wird und die in Längsrichtung verlaufenden Führungen (3, 4) verbindet.

6. Fadenanker nach einem der Ansprüche 1 bis 3, **dadurch gekennzeichnet, dass** die in Längsrichtung verlaufenden Führungen (3, 4) aus zwei in Längsrichtung parallel zum Ankerkörper (1) verlaufenden und den Ankerkörper durchdringenden Löchern bestehen, wobei der Umkehrabschnitt (5) von einer an der Oberfläche des Kopfes (1a) des Ankerkörpers ausgebildeten Nut gebildet wird und die in Längsrichtung verlaufenden Führungen (3, 4) verbindet.

7. Fadenanker nach einem der vorhergehenden Ansprüche, **dadurch gekennzeichnet, dass** die äußere Oberfläche des Ankerkörpers (1) mit einem Satz von Vorsprüngen ausgestattet ist, vorzugsweise einem Schraubengewinde (2), das im Wesentlichen die gleiche Länge wie die Längsseite des Ankerkörpers (1) besitzt.

8. Fadenanker nach einem der vorhergehenden Ansprüche, **dadurch gekennzeichnet, dass** der Ankerkörper (1) mit einer an dessen Basis (1b) ausgebildeten Verbindungseinrichtung (6) zum Verbinden des Fadenankers mit einer externen Vorrichtung zum Einsetzen des Fadenankers in den Knochen ausgestattet ist.

9. Fadenanker nach Anspruch 7 oder 8, **dadurch gekennzeichnet, dass** die Verbindungseinrichtung (6) eine Buchse oder dergleichen zum Verbinden des Fadenankers mit einem Werkzeugschlüssel oder dergleichen aufweist.

10. Fadenanker nach einem der vorhergehenden Ansprüche, **dadurch gekennzeichnet, dass** der Ankerkörper (1) aus einem biologisch abbaubaren Material hergestellt ist.

11. Fadenanker nach einem der vorhergehenden Ansprüche, **dadurch gekennzeichnet, dass** sich der Faden nicht über den Kopf (1a) des Ankerkörpers hinaus erstreckt.

## Revendications

1. Dispositif de fixation de suture en une seule pièce destiné à fixer une suture (7) dans un os, dans lequel un trou destiné audit dispositif de fixation de suture est formé dans l'os, ledit dispositif de fixation de suture comprenant un corps de fixation allongé (1) ayant une tête (1a) et une base (1b), ledit corps (1) étant muni d'un canal de suture (K) composé de deux guides longitudinaux allongés (3, 4) sensiblement alignés avec (la) direction longitudinale du corps (1) du dispositif de fixation et d'une partie rotative (5) qui combine les guides longitudinaux au niveau de la tête du corps du dispositif de fixation (1a), **caractérisé en ce que** ladite partie rotative (5) est formée sensiblement de sorte à être alignée avec la dimension longitudinale du dispositif de fixation de suture, dans lequel le point de rotation au niveau de la tête e la partie rotative (5) est situé à une distance longitudinale des jonctions (5a, 5b) des guides longitudinaux (3, 4) et de la partie rotative (5), dans lequel les guides longitudinaux et la partie rotative ont une forme de rainure et sont situés sur la surface extérieure du dispositif de fixation de suture, ou mes guides longitudinaux sont des canaux formés à l'intérieur du corps du dispositif de fixation.

2. Dispositif de fixation de suture selon la revendication 1, **caractérisé en ce que** l'angle d'altération de la partie rotative (5) entre les jonctions (5a, 5b) est continu, et **en ce que** la partie rotative (5) possède une configuration courbée.

3. Dispositif de fixation de suture selon la revendication 1, **caractérisé en ce que** chaque angle d'altération de la partie rotative (5) entre les jonctions (5a, 5b) est inférieur à 90°.

4. Dispositif de fixation de suture selon l'une quelconque des revendications précédentes, **caractérisé en ce que** les guides longitudinaux (3, 4) se composent de deux trous qui sont ouverts au niveau de la base (1b) du corps du dispositif de fixation et sont positionnés à l'intérieur du corps du dispositif de fixation (1), lesdits trous étant reliés ensemble à leurs extrémités au niveau de la tête du corps du dispositif de fixation (1a) qui restent à l'intérieur du corps du dispositif de fixation (1) à l'aide d'une partie rotative tubulaire (5) qui est située à l'intérieur du corps du dispositif de fixation (1).

5. Dispositif de fixation de suture selon l'une quelconque des revendications 1 à 3, **caractérisé en ce que** les guides longitudinaux (3, 4) se composent de deux rainures formées sur la surface du corps du dispositif de fixation (1) et s'étendant parallèlement à la direction longitudinale du corps du dispositif de fixation (1) entre la base (1b) et la tête (1a) du corps du dispositif de fixation, sensiblement vers les côtés opposés du corps du dispositif de fixation (1), dans lequel la partie rotative (5) est composée de la rainure formée sur la surface de la tête (1a) du corps du dispositif de fixation et reliant les guides longitudinaux (3, 4).

6. Dispositif de fixation de suture selon l'une quelconque des revendications 1 à 3, **caractérisé en ce que** les guides longitudinaux (3, 4) se composent de deux trous longitudinalement parallèles au corps du dispositif de fixation (1) et pénétrant dans ledit corps du dispositif de fixation, dans lequel la partie rotative (5) est composée d'une rainure formée sur la surface de la tête (1a) du corps du dispositif de fixation et reliant les guides longitudinaux (3, 4).

7. Dispositif de fixation de suture selon l'une quelconque des revendications précédentes, **caractérisé en ce que** la surface extérieure du corps du dispositif de fixation (1) est munie d'un ensemble de projections, et avantageusement d'un filetage à vis (2), qui est sensiblement de la même longueur que le côté longitudinal du corps du dispositif de fixation (1).

8. Dispositif de fixation de suture selon l'une quelconque des revendications précédentes, **caractérisé en ce que** le corps du dispositif de fixation (1) est muni de moyens de raccordement (6) formés au niveau de la base (1b) de celui-ci afin de relier le dispositif de fixation de suture à un dispositif externe destiné à fixer le dispositif de fixation de suture dans l'os.

9. Dispositif de fixation de suture selon les revendications 7 et 8, **caractérisé en ce que** les moyens de raccordement (6) comprennent un manchon ou similaire destiné à relier le dispositif de fixation de suture à une clé ou similaire.

10. Dispositif de fixation de suture selon l'une quelconque des revendications précédentes, **caractérisé en ce que** le corps du dispositif de fixation (1) est fabriqué en matériau biodégradable.

11. Dispositif de fixation de suture selon l'une quelconque des revendications précédentes, **caractérisé en ce que** la suture ne s'étend pas au-delà de la tête (1a) du corps de fixation.
